Europäisches Patentamt

European Patent Office

Office européen des brevets

(19)

(11) Publication number: **0 278 541**
A1

# EUROPEAN PATENT APPLICATION

(21) Application number: 88200045.8

(22) Date of filing: 14.01.88

(51) Int. Cl.4: **C12N 15/00** , A61K 39/225 , C12P 21/02 , C12N 1/20

(30) Priority: 16.01.87 NL 8700095

(43) Date of publication of application:
17.08.88 Bulletin 88/33

(84) Designated Contracting States:
AT BE CH DE ES FR GB GR IT LI LU NL SE

(71) Applicant: DUPHAR INTERNATIONAL RESEARCH B.V
C.J. van Houtenlaan 36
NL-1381 CP Weesp(NL)

(72) Inventor: Jacobs, Catharina E.
c/o OCTROOIBUREAU ZOAN B.V. P.O. Box 140
NL-1380 AC Weesp(NL)
Inventor: Spaan, Wilhelmus J. M.
c/o OCTROOIBUREAU ZOAN B.V. P.O. Box 140
NL-1380 AC Weesp(NL)

(74) Representative: Muis, Maarten et al
OCTROOIBUREAU ZOAN B.V. P.O. Box 140
NL-1380 AC Weesp(NL)

(54) **Antigenically active proteins and peptides, and transmissible gastro-enteritis virus TGEV vaccines.**

(57) The invention relates to new antigenic proteins which can be used to stimulate the immunity of pigs against transmissible gastro-enteritis virus (TGEV)-vaccines, and to fragments or derivatives thereof. The invention also relates to vaccines which comprise such antigenic proteins or peptides.

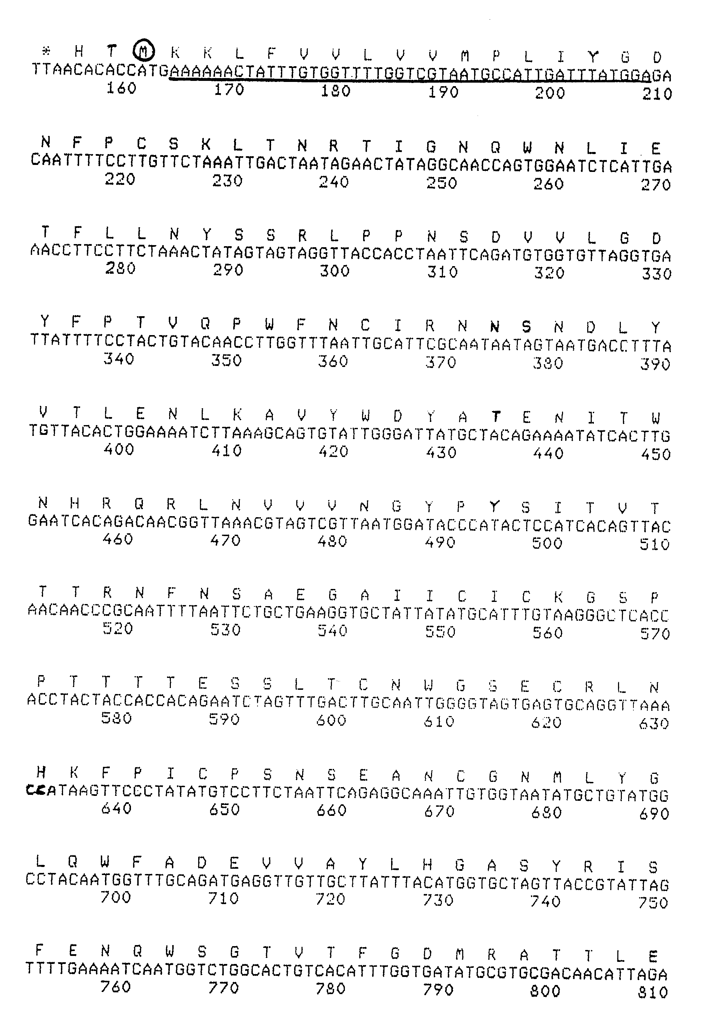

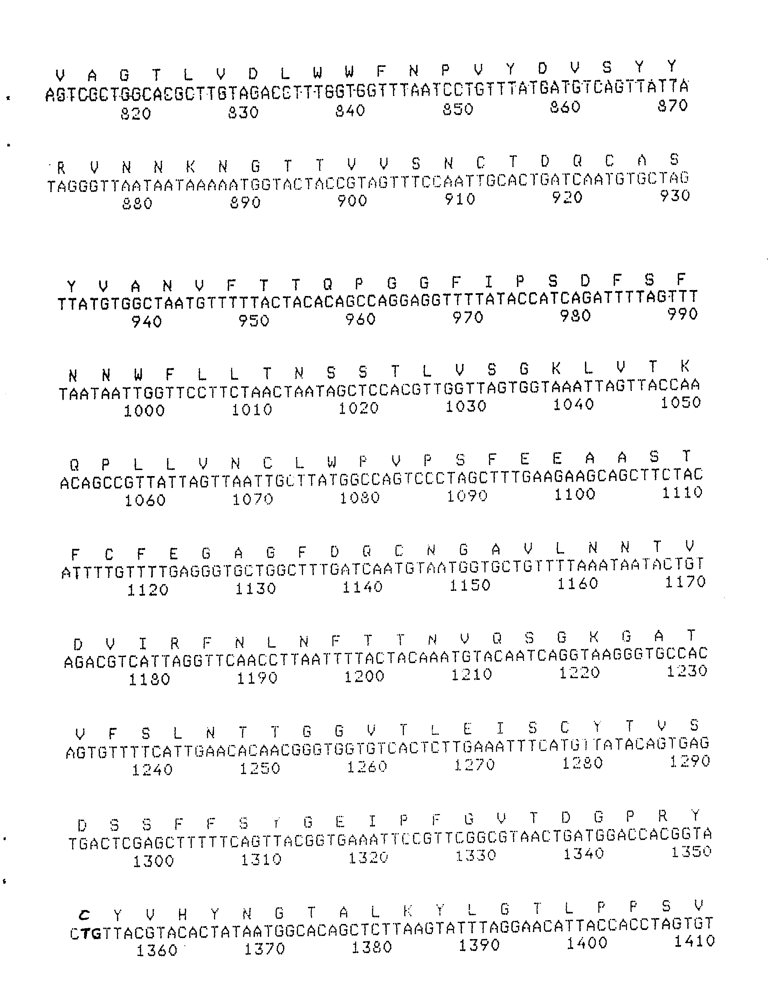

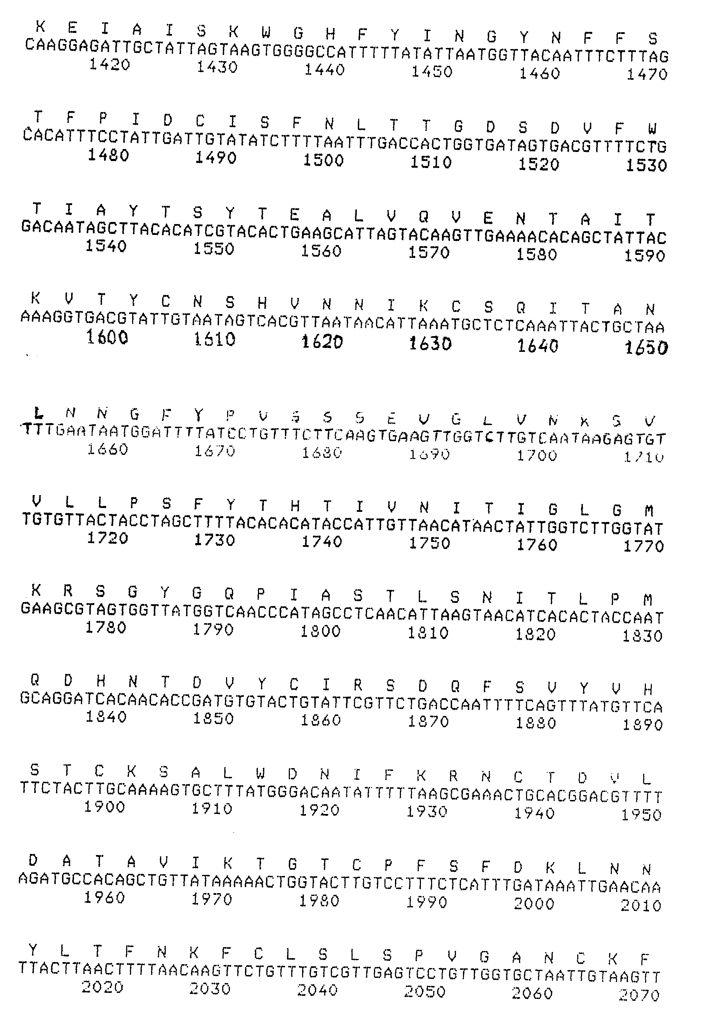

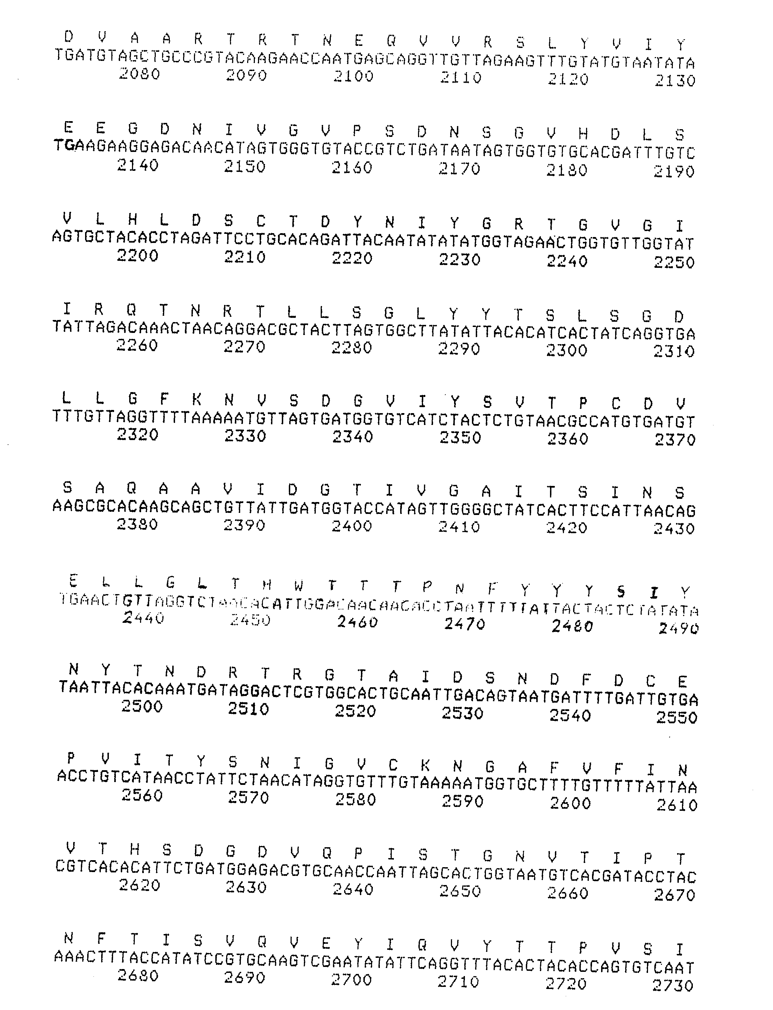

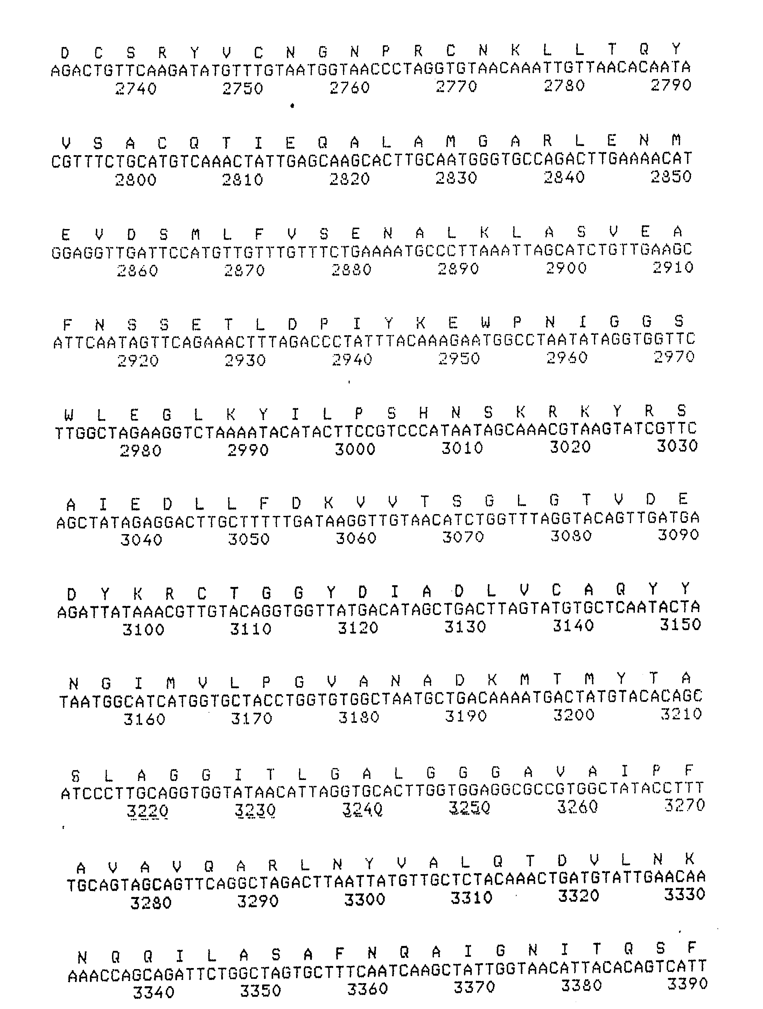

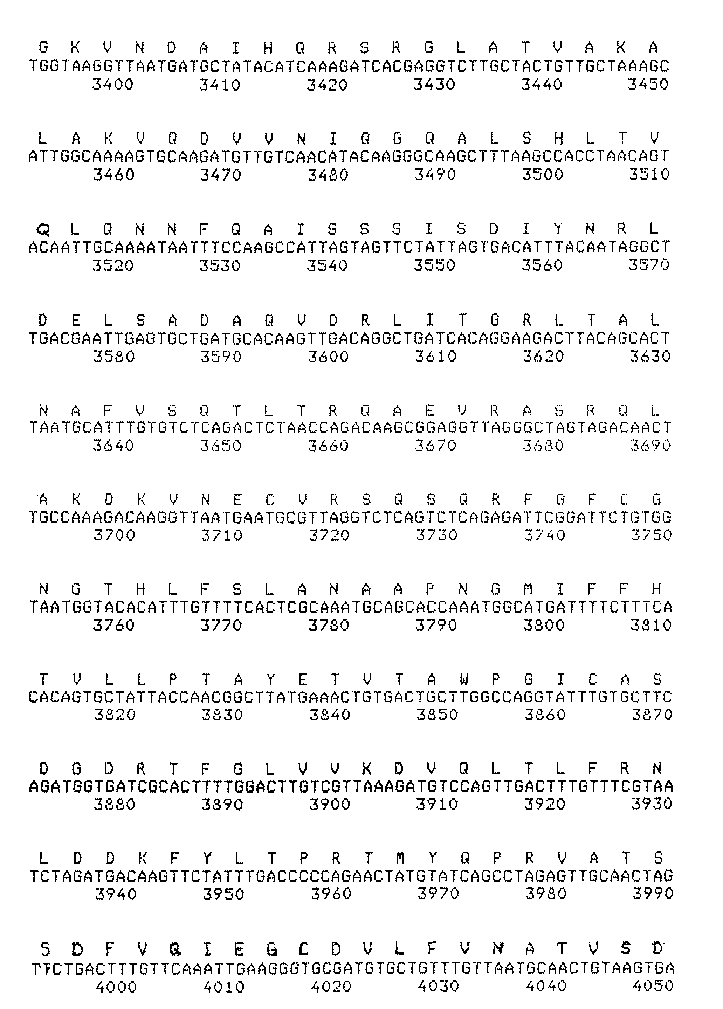

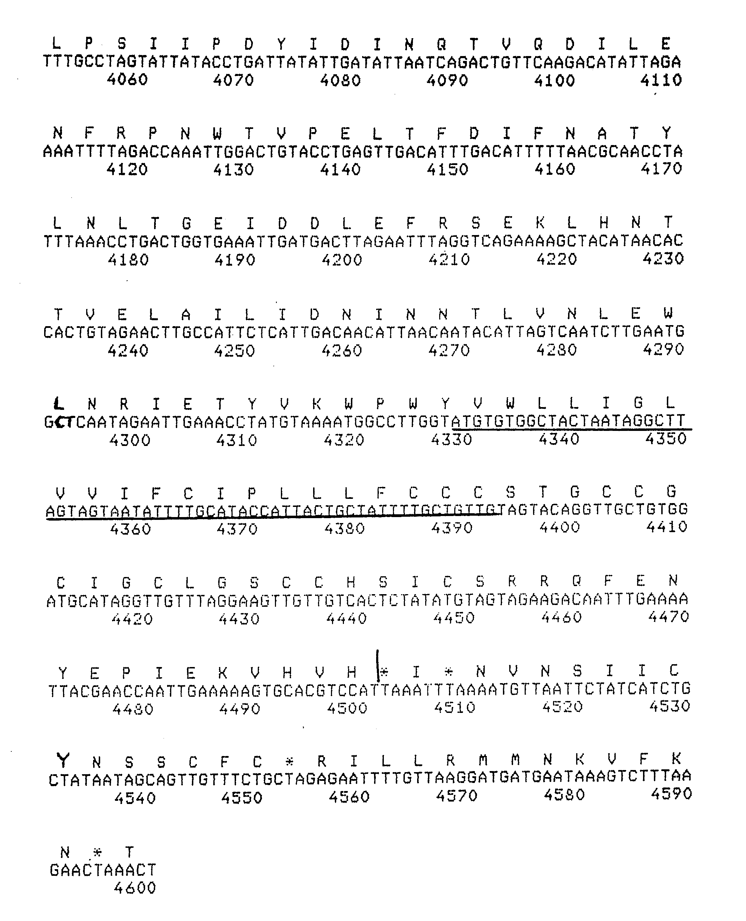

The nucleotide sequence and amine acid sequence of a gene coding for protein of an TGEV-strain are indicated in figure 1.

EP 0 278 541 A1

# New antigenically active proteins and peptides, and transmissible gastro-enteritis virus (TGEV)-vaccines.

The invention relates to a new antigenic protein which is capable of stimulating the immunity of pigs against transmissible gastro-enteritis virus (TGEV), and to fragments or derivatives thereof. The invention further relates to vaccines which comprises such an antigenic protein or peptide.

Transmissible gastro-enteritis is an infection disease of the intestine in pigs causing high mortality in piglings younger than 2 weeks and hence great economic losses to the pig breeders. Inspite of the existance of various types of vaccines, the problem of TGE has not been solved effectively. Therefore, the development of new, better vaccines against TGE is urgently necessary.

The cause of TGE is a virus which belongs to the group or corona viruses. Corona viruses are positive single-stranded RNA viruses having a lipid envelope. The TGEV genome RNA has a length of approximately 23,600 bases. The virus particle (virion) comprises three important proteins, namely the peplomeric protein (S), the nucleocapside protein (N), and the matrix protein (M).

The characteristic protrusions at the surface of the virion are formed by the peplomeric protein (S). Each peplomer is formed by a glycopolypeptide. The protein is fixed in the membrane by means of a terminal membrane anchor sequence. It is known from literature that antibodies generated against the peplomeric protein of the said virus, provide protection to the pig.

According to the present invention the nucleotide sequence and the amino acid sequence of the TGEV Purdue strain were determined.

The invention therefore relates to proteins and fragments thereof which comprise at least one of the areas which play a part in the protection against TGE infections.

Theoretically, such fragments can be used as such as an antigen for vaccinating against TGE. However, it is to be preferred to couple said fragments to a suitable carrier.

The peptide fragments or proteins according to the invention which comprise at least one antigenically active peptide fragment can be prepared according to methods known for the preparation of peptides and proteins.

First of all, the peptides can be prepared synthetically by means of known techniques starting from the individual amino acids or smaller peptide fragments.

The peptides can also be obtained biosynthetically using recombinant DNA techniques and expression systems, for example, by:

a) transformation of host cells with an expression vector which comprises a DNA, coding for an (the) antigenic determinant(s) (peptides in general);

b) expressing the genome inserted in the expression vector;

c) harvesting the cell culture, and

d) separating the synthesised peptide (protein).

The invention therefore also relates to a method of preparing a DNA molecule which codes for peptides according to the invention. Such a method comprises the folllowing steps:

a) isloation of TGE single-stranded RNA;

b) synthesis of a cDNA strand complementary to the RNA strand mentioned in a); and

c) removal of the RNA molecule and synthesis of a second cDNA strand, using the first cDNA strand as a template.

The double-stranded DNA obtained in this manner may be incorporated in an expression vector in known manner so that a recombinant expression vector is formed. This vector may be introduced into a suitable host cell, for example, by transformation.

The invention will now be described in greater detail with reference to the ensuing specific example.

## EXAMPLE

### a) Virus and tissue culture.

TGEV strain Purdue was purified twice by means of pricking of a plaque on PD5 cells. Culture conditions were described previously in L. Jacobs et al, J. Virol. 57; (1986); 1010-15.

### b) Isolation of subgenomic mRNA's

PD5 cells ($9 \times 10^7$) were infected with an infection multiplicity of 15. Seven hours after infection actinomycine D was added and 10 hours after infection the cells well lysed and the RNA extracted, as described previously by W.J.M. Spaan et al., Virol. 108 (1981); 424-434. The RNA (560 $\mu$g) was dissolved in a buffer having a high salt concentration (10 mM Tris-HCl pH 7.5, 0.05% SDS, 500 mM NaCl) and transferred to an oligo(dT)-cellulose column for a selection on poly(A)+ RNA. After elution with 10 mM Tris-HCl pH 7.5the RNA was concentrated by precipitation with ethanol and fractionated by means of an isokinetic sucrose gradient (B.A.M. van der Zeijst et al., in G.D. Fasman (Ed.), Handbook of biochemistry and molecular biology, 3rd ed. Physical and Chemical data. Vol. 1 CRC Press Inc., Boca Raton, Fla., (1976), pp. 426-519). Before providing the RNA on the gradient it was dissolved in 50 mM Tris-HCl pH 7.4, 10 mM LiCl, 1% SDS and heated at 56°C for 1 minute. $^{32}$P-labelled TGEV mRNA's were added as markers. The identification and translation of TGEV mRNA's is described by Jacobs et al., 1986.

c) Cloning of TGEV peplomer (E2) gene.

RNA3 obtained after purification over the sucrose gradient was used for the synthesis of cDNA (carried out as described by Gubler and Hoffman (Gene, 25 (1983), 263-269). Calf-thymus pentamers were used as primers and methyl mercury hydroxide for denaturing the RNA. The double-stranded cDNA was elongated with dC residues and then cloned in a pUC9 vector elongated with dG residues. The DNA was used for the transformation of E. coli strain JM109 as described by D. Hanahan et al., J. Mol. Biol., 166, (1983), 557-580. In this manner a cDNA library of approximately 900 transformants was obtained. The plasmides comprise insertions having a length up to 5kb.

d) Selection of E2-specific recombinants.

E2-specific recombinants could be identified by using the homology between TGEV and FIPV. Two restriction fragments from the E2 gene of FIPV were labelled with $^{32}$P-dATP by means of nick translation and were used for the selection of the recombinants. Twenty-four recombinants hybridised with probe 2, eight with probe 1 and two recombinants hybridised with both probes. A restriction map of three recombinants was made. The recombinants which represent the whole peplomer gene were used for sequence analysis. Restriction fragments were separated in an agarose gel and isolated by means of an NA45 membrane (Schleicher and Schuell) and cloned in M13 vectors (mp8 and/or mp19). Sequence analysis was carried out according to the dideoxy-nucleotide termination method of Sanger et al., Proc. Nath. Acad. Sci. USA 74, (1977), 721-732. Both the universal M13 primer and E2-specific primers were used. The data were analysed by means of a DEC20/60 computer and the programs of Staden, Nucl. Acid. Res. 10, (1982), 4731-4751.

e) Nucleotide sequence.

The nucleotide sequence of the peplomer gene of TGEV (Purdue strain) is shown in figure 1. The sequence comprises an open reading frame of 4347 nucelotides with a coding capacity for a precursor protein of 1449 amino acids (nucleotide position 155-4502). Upstream of the ATG codon and downstream of first stop codon a recurring sequence (ACTAAACT) is present. This "intergenic" sequence also flanks the E2 gene of FIPV and has also been found upstream of the nucleocapsid gene of TGEV and MHV.

f) Amino acid sequence.

The ATG translation initiation codon (position 161) is succeeded by 15 amino acids which probably form the cleavable signal peptide, two hydrophilic amino acids succeeded by 13 hydrophobic amino acids. The extreme carboxy terminal part, position 4319-4393 (25 amino acids) comprises a hydrophobic region which presumable serves as a transmembrane anchor, both regions are underlined in Figure 1.

**Claims**

1. A gene which codes for protein of transmissible gastro-enteritis virus (TGEV), characterized by the nucelotide sequence as shown in Figure 1.

2. A protein, characterized by the amino acid sequence as shown in Figure 1, or a part thereof.

3. An immunogen, characterized in that it comprises a protein or peptide as claimed in Claim 2, whether or nor coupled to a carrier.

4. A vaccine, characterized in that it comprises an immunogen as claimed in Claim 3.

5. A method of preparing a protein or peptide as claimed in Claim 2, characterized in that

a) the protein or peptide is synthesised in a manner known per su from individual amino acids and/or by coupling smaller peptides; or

b) a host cell is transformed with an expression vector which comprises a DNA coding for a protein or peptide as claimed in Claim 2, and the genome introduced in the expression vector is expressed.

6. A DNA molecule which comprises a nucleotide sequence which codes for a protein or peptide as claimed in Claim 2, or which codes for a polypeptide which comprises a protein or peptide as claimed in Claim 2.

7. A recombinant DNA expression vector which expresses the whole protein or peptide or a part thereof as defined in Claim 2, comprising an operon with initiator sequences and terminator sequence and a nucleotide sequence which codes for the said protein or peptide, the said nucleotide sequence being situated between the initiator sequence and the terminator sequence of the operon.

8. A host cell which comprises a recombinant DNA expression vector and/or DNA molecule as claimed in Claim 6 or 7.

9. A method of preparing a DNA molecule which codes for a protein or peptide as claimed in Claim 2, characterized in that

a) single-stranded TGEV-RNA is isolated;

b) a cDNA strand complementary to said RNA strand is synthesised;

c) the RNA strand is removed and a second strand of cDNA is synthesised with the first cDNA as a template.

```
 *  H  T (M) K  K  L  F  V  V  L  V  V  M  P  L  I  Y  G  D
TTAACACCATGAAAAAACTATTTGTGGTTTTGGTCGTAATGCCATTGATTTATGGAGA

    N  F  P  C  S  K  L  T  N  R  T  I  G  N  Q  W  N  L  I  E
CAATTTTCCTTGTTCTAAATTGACTAATAGAACTATAGGCAACCAGTGGAATCTCATTGA

    T  F  L  L  N  Y  S  S  R  L  P  P  N  S  D  V  V  L  G  D
AACCTTCCTTCTAAACTATAGTAGTAGGTTACCACCTAATTCAGATGTGGTGTTAGGTGA

    Y  F  P  T  V  Q  P  W  F  N  C  I  R  N  N  S  N  D  L  Y
TTATTTTCCTACTGTACAACCTTGGTTTAATTGCATTCGCAATAATAGTAATGACCTTTA

    V  T  L  E  N  L  K  A  V  Y  W  D  Y  A  T  E  N  I  T  W
TGTTACACTGGAAAATCTTAAAGCAGTGTATTGGGATTATGCTACAGAAAATATCACTTG

    N  H  R  Q  R  L  N  V  V  V  N  G  Y  P  Y  S  I  T  V  T
GAATCACAGACAACGGTTAAACGTAGTCGTTAATGGATACCCATACTCCATCACAGTTAC

    T  T  R  N  F  N  S  A  E  G  A  I  I  C  I  C  K  G  S  P
AACAACCCGCAATTTTAATTCTGCTGAAGGTGCTATTATATGCATTTGTAAGGGCTCACC

    P  T  T  T  T  E  S  S  L  T  C  N  W  G  S  E  C  R  L  N
ACCTACTACCACCACAGAATCTAGTTTGACTTGCAATTGGGGTAGTGAGTGCAGGTTAAA

    H  K  F  P  I  C  P  S  N  S  E  A  N  C  G  N  M  L  Y  G
CCATAAGTTCCCTATATGTCCTTCTAATTCAGAGGCAAATTGTGGTAATATGCTGTATGG

    L  Q  W  F  A  D  E  V  V  A  Y  L  H  G  A  S  Y  R  I  S
CCTACAATGGTTTGCAGATGAGGTTGTTGCTTATTTACATGGTGCTAGTTACCGTATTAG

    F  E  N  Q  W  S  G  T  V  T  F  G  D  M  R  A  T  T  L  E
TTTTGAAAATCAATGGTCTGGCACTGTCACATTTGGTGATATGCGTGCGACAACATTAGA
```

DUPHAR INTERNATIONAL RESEARCH B.V.          DIR 0389

```
     V   A   G   T   L   V   D   L   W   W   F   N   P   V   Y   D   V   S   Y   Y
    AGTCGCTGGCACGCTTGTAGACCTTTGGTGGTTTAATCCTGTTTATGATGTCAGTTATTA
         820       830       840       850       860       870

      R   V   N   N   K   N   G   T   T   V   V   S   N   C   T   D   Q   C   A   S
    TAGGGTTAATAATAAAAATGGTACTACCGTAGTTTCCAATTGCACTGATCAATGTGCTAG
         880       890       900       910       920       930


      Y   V   A   N   V   F   T   T   Q   P   G   G   F   I   P   S   D   F   S   F
    TTATGTGGCTAATGTTTTTACTACACAGCCAGGAGGTTTTATACCATCAGATTTTAGTTT
         940       950       960       970       980       990


      N   N   W   F   L   L   T   N   S   S   T   L   V   S   G   K   L   V   T   K
    TAATAATTGGTTCCTTCTAACTAATAGCTCCACGTTGGTTAGTGGTAAATTAGTTACCAA
        1000      1010      1020      1030      1040      1050


      Q   P   L   L   V   N   C   L   W   P   V   P   S   F   E   E   A   A   S   T
    ACAGCCGTTATTAGTTAATTGCTTATGGCCAGTCCCTAGCTTTGAAGAAGCAGCTTCTAC
        1060      1070      1080      1090      1100      1110


      F   C   F   E   G   A   G   F   D   Q   C   N   G   A   V   L   N   N   T   V
    ATTTTGTTTTGAGGGTGCTGGCTTTGATCAATGTAATGGTGCTGTTTAAATAATACTGT
        1120      1130      1140      1150      1160      1170


      D   V   I   R   F   N   L   N   F   T   T   N   V   Q   S   G   K   G   A   T
    AGACGTCATTAGGTTCAACCTTAATTTTACTACAAATGTACAATCAGGTAAGGGTGCCAC
        1180      1190      1200      1210      1220      1230


      V   F   S   L   N   T   T   G   G   V   T   L   E   I   S   C   Y   T   V   S
    AGTGTTTTCATTGAACACAACGGGTGGTGTCACTCTTGAAATTTCATGTTATACAGTGAG
        1240      1250      1260      1270      1280      1290


      D   S   S   F   F   S   Y   G   E   I   P   F   G   V   T   D   G   P   R   Y
    TGACTCGAGCTTTTTCAGTTACGGTGAAATTCCGTTCGGCGTAACTGATGGACCACGGTA
        1300      1310      1320      1330      1340      1350


      C   Y   V   H   Y   N   G   T   A   L   K   Y   L   G   T   L   P   P   S   V
    CTGTTACGTACACTATAATGGCACAGCTCTTAAGTATTTAGGAACATTACCACCTAGTGT
        1360      1370      1380      1390      1400      1410
```

DUPHAR INTERNATIONAL RESEARCH B.V.                     DIR 0389

```
     K   E   I   A   I   S   K   W   G   H   F   Y   I   N   G   Y   N   F   F   S
   CAAGGAGATTGCTATTAGTAAGTGGGGCCATTTTTATATTAATGGTTACAATTTCTTTAG
       1420        1430        1440        1450        1460        1470


     T   F   P   I   D   C   I   S   F   N   L   T   T   G   D   S   D   V   F   W
   CACATTTCCTATTGATTGTATATCTTTTAATTTGACCACTGGTGATAGTGACGTTTTCTG
       1480        1490        1500        1510        1520        1530


     T   I   A   Y   T   S   Y   T   E   A   L   V   Q   V   E   N   T   A   I   T
   GACAATAGCTTACACATCGTACACTGAAGCATTAGTACAAGTTGAAAACACAGCTATTAC
       1540        1550        1560        1570        1580        1590


     K   V   T   Y   C   N   S   H   V   N   N   I   K   C   S   Q   I   T   A   N
   AAAGGTGACGTATTGTAATAGTCACGTTAATAACATTAAATGCTCTCAAATTACTGCTAA
       1600        1610        1620        1630        1640        1650


     L   N   N   G   F   Y   P   V   S   S   S   E   V   G   L   V   N   K   S   V
   TTTGAATAATGGATTTTATCCTGTTTCTTCAAGTGAAGTTGGTCTTGTCAATAAGAGTGT
       1660        1670        1680        1690        1700        1710


     V   L   L   P   S   F   Y   T   H   T   I   V   N   I   T   I   G   L   G   M
   TGTGTTACTACCTAGCTTTTACACACATACCATTGTTAACATAACTATTGGTCTTGGTAT
       1720        1730        1740        1750        1760        1770


     K   R   S   G   Y   G   Q   P   I   A   S   T   L   S   N   I   T   L   P   M
   GAAGCGTAGTGGTTATGGTCAACCCATAGCCTCAACATTAAGTAACATCACACTACCAAT
       1780        1790        1800        1810        1820        1830


     Q   D   H   N   T   D   V   Y   C   I   R   S   D   Q   F   S   V   Y   V   H
   GCAGGATCACAACACCGATGTGTACTGTATTCGTTCTGACCAATTTTCAGTTTATGTTCA
       1840        1850        1860        1870        1880        1890


     S   T   C   K   S   A   L   W   D   N   I   F   K   R   N   C   T   D   V   L
   TTCTACTTGCAAAAGTGCTTTATGGGACAATATTTTTAAGCGAAACTGCACGGACGTTTT
       1900        1910        1920        1930        1940        1950


     D   A   T   A   V   I   K   T   G   T   C   P   F   S   F   D   K   L   N   N
   AGATGCCACAGCTGTTATAAAAACTGGTACTTGTCCTTCTCATTTGATAAATTGAACAA
       1960        1970        1980        1990        2000        2010


     Y   L   T   F   N   K   F   C   L   S   L   S   P   V   G   A   N   C   K   F
   TTACTTAACTTTTAACAAGTTCTGTTTGTCGTTGAGTCCTGTTGGTGCTAATTGTAAGTT
       2020        2030        2040        2050        2060        2070
```

DUPHAR INTERNATIONAL RESEARCH B.V.                    DIR 0389

```
        D   V   A   A   R   T   R   T   N   E   Q   V   V   R   S   L   Y   V   I   Y
      TGATGTAGCTGCCCGTACAAGAACCAATGAGCAGGTTGTTAGAAGTTTGTATGTAATATA
         2080        2090        2100        2110        2120        2130


        E   E   G   D   N   I   V   G   V   P   S   D   N   S   G   V   H   D   L   S
      TGAAGAAGGAGACAACATAGTGGGTGTACCGTCTGATAATAGTGGTGTGCACGATTTGTC
         2140        2150        2160        2170        2180        2190


        V   L   H   L   D   S   C   T   D   Y   N   I   Y   G   R   T   G   V   G   I
      AGTGCTACACCTAGATTCCTGCACAGATTACAATATATATGGTAGAACTGGTGTTGGTAT
         2200        2210        2220        2230        2240        2250


        I   R   Q   T   N   R   T   L   L   S   G   L   Y   Y   T   S   L   S   G   D
      TATTAGACAAACTAACAGGACGCTACTTAGTGGCTTATATTACACATCACTATCAGGTGA
         2260        2270        2280        2290        2300        2310


        L   L   G   F   K   N   V   S   D   G   V   I   Y   S   V   T   P   C   D   V
      TTTGTTAGGTTTTAAAAATGTTAGTGATGGTGTCATCTACTCTGTAACGCCATGTGATGT
         2320        2330        2340        2350        2360        2370


        S   A   Q   A   A   V   I   D   G   T   I   V   G   A   I   T   S   I   N   S
      AAGCGCACAAGCAGCTGTTATTGATGGTACCATAGTTGGGGCTATCACTTCCATTAACAG
         2380        2390        2400        2410        2420        2430


        E   L   L   G   L   T   H   W   T   T   T   P   N   F   Y   Y   Y   S   I   Y
      TGAACTGTTAGGTCTAACACATTGGACAACAACACCTAATTTTTATTACTACTCTATATA
         2440        2450        2460        2470        2480        2490


        N   Y   T   N   D   R   T   R   G   T   A   I   D   S   N   D   F   D   C   E
      TAATTACACAAATGATAGGACTCGTGGCACTGCAATTGACAGTAATGATTTTGATTGTGA
         2500        2510        2520        2530        2540        2550


        P   V   I   T   Y   S   N   I   G   V   C   K   N   G   A   F   V   F   I   N
      ACCTGTCATAACCTATTCTAACATAGGTGTTTGTAAAAATGGTGCTTTTGTTTTTATTAA
         2560        2570        2580        2590        2600        2610


        V   T   H   S   D   G   D   V   Q   P   I   S   T   G   N   V   T   I   P   T
      CGTCACACATTCTGATGGAGACGTGCAACCAATTAGCACTGGTAATGTCACGATACCTAC
         2620        2630        2640        2650        2660        2670


        N   F   T   I   S   V   Q   V   E   Y   I   Q   V   Y   T   T   P   V   S   I
      AAACTTTACCATATCCGTGCAAGTCGAATATATTCAGGTTTACACTACACCAGTGTCAAT
         2680        2690        2700        2710        2720        2730
```

DUPHAR INTERNATIONAL RESEARCH B.V.                    DIR 0389

```
      D   C   S   R   Y   V   C   N   G   N   P   R   C   N   K   L   L   T   Q   Y
     AGACTGTTCAAGATATGTTTGTAATGGTAACCCTAGGTGTAACAAATTGTTAACACAATA
        2740      2750      2760      2770      2780      2790


      V   S   A   C   Q   T   I   E   Q   A   L   A   M   G   A   R   L   E   N   M
     CGTTTCTGCATGTCAAACTATTGAGCAAGCACTTGCAATGGGTGCCAGACTTGAAAACAT
        2800      2810      2820      2830      2840      2850


      E   V   D   S   M   L   F   V   S   E   N   A   L   K   L   A   S   V   E   A
     GGAGGTTGATTCCATGTTGTTTGTTTCTGAAAATGCCCTTAAATTAGCATCTGTTGAAGC
        2860      2870      2880      2890      2900      2910


      F   N   S   S   E   T   L   D   P   I   Y   K   E   W   P   N   I   G   G   S
     ATTCAATAGTTCAGAAACTTTAGACCCTATTTACAAAGAATGGCCTAATATAGGTGGTTC
        2920      2930      2940      2950      2960      2970


      W   L   E   G   L   K   Y   I   L   P   S   H   N   S   K   R   K   Y   R   S
     TTGGCTAGAAGGTCTAAAATACATACTTCCGTCCCATAATAGCAAACGTAAGTATCGTTC
        2980      2990      3000      3010      3020      3030


      A   I   E   D   L   L   F   D   K   V   V   T   S   G   L   G   T   V   D   E
     AGCTATAGAGGACTTGCTTTTTGATAAGGTTGTAACATCTGGTTTAGGTACAGTTGATGA
        3040      3050      3060      3070      3080      3090


      D   Y   K   R   C   T   G   G   Y   D   I   A   D   L   V   C   A   Q   Y   Y
     AGATTATAAACGTTGTACAGGTGGTTATGACATAGCTGACTTAGTATGTGCTCAATACTA
        3100      3110      3120      3130      3140      3150


      N   G   I   M   V   L   P   G   V   A   N   A   D   K   M   T   M   Y   T   A
     TAATGGCATCATGGTGCTACCTGGTGTGGCTAATGCTGACAAAATGACTATGTACACAGC
        3160      3170      3180      3190      3200      3210


      S   L   A   G   G   I   T   L   G   A   L   G   G   G   A   V   A   I   P   F
     ATCCCTTGCAGGTGGTATAACATTAGGTGCACTTGGTGGAGGCGCCGTGGCTATACCTTT
        3220      3230      3240      3250      3260      3270


      A   V   A   V   Q   A   R   L   N   Y   V   A   L   Q   T   D   V   L   N   K
     TGCAGTAGCAGTTCAGGCTAGACTTAATTATGTTGCTCTACAAACTGATGTATTGAACAA
        3280      3290      3300      3310      3320      3330


      N   Q   Q   I   L   A   S   A   F   N   Q   A   I   G   N   I   T   Q   S   F
     AAACCAGCAGATTCTGGCTAGTGCTTTCAATCAAGCTATTGGTAACATTACACAGTCATT
        3340      3350      3360      3370      3380      3390
```

DUPHAR INTERNATIONAL RESEARCH B.V.                    DIR 0389

```
        G   K   V   N   D   A   I   H   Q   R   S   R   G   L   A   T   V   A   K   A
       TGGTAAGGTTAATGATGCTATACATCAAAGATCACGAGGTCTTGCTACTGTTGCTAAAGC
          3400      3410      3420      3430      3440      3450


        L   A   K   V   Q   D   V   V   N   I   Q   G   Q   A   L   S   H   L   T   V
       ATTGGCAAAAGTGCAAGATGTTGTCAACATACAAGGGCAAGCTTTAAGCCACCTAACAGT
          3460      3470      3480      3490      3500      3510


        Q   L   Q   N   N   F   Q   A   I   S   S   S   I   S   D   I   Y   N   R   L
       ACAATTGCAAAATAATTTCCAAGCCATTAGTAGTTCTATTAGTGACATTTACAATAGGCT
          3520      3530      3540      3550      3560      3570


        D   E   L   S   A   D   A   Q   V   D   R   L   I   T   G   R   L   T   A   L
       TGACGAATTGAGTGCTGATGCACAAGTTGACAGGCTGATCACAGGAAGACTTACAGCACT
          3580      3590      3600      3610      3620      3630


        N   A   F   V   S   Q   T   L   T   R   Q   A   E   V   R   A   S   R   Q   L
       TAATGCATTTGTGTCTCAGACTCTAACCAGACAAGCGGAGGTTAGGGCTAGTAGACAACT
          3640      3650      3660      3670      3680      3690


        A   K   D   K   V   N   E   C   V   R   S   Q   S   Q   R   F   G   F   C   G
       TGCCAAAGACAAGGTTAATGAATGCGTTAGGTCTCAGTCTCAGAGATTCGGATTCTGTGG
          3700      3710      3720      3730      3740      3750


        N   G   T   H   L   F   S   L   A   N   A   A   P   N   G   M   I   F   F   H
       TAATGGTACACATTTGTTTTCACTCGCAAATGCAGCACCAAATGGCATGATTTTCTTTCA
          3760      3770      3780      3790      3800      3810


        T   V   L   L   P   T   A   Y   E   T   V   T   A   W   P   G   I   C   A   S
       CACAGTGCTATTACCAACGGCTTATGAAACTGTGACTGCTTGGCCAGGTATTTGTGCTTC
          3820      3830      3840      3850      3860      3870


        D   G   D   R   T   F   G   L   V   V   K   D   V   Q   L   T   L   F   R   N
       AGATGGTGATCGCACTTTTGGACTTGTCGTTAAAGATGTCCAGTTGACTTTGTTTCGTAA
          3880      3890      3900      3910      3920      3930


        L   D   D   K   F   Y   L   T   P   R   T   M   Y   Q   P   R   V   A   T   S
       TCTAGATGACAAGTTCTATTTGACCCCCAGAACTATGTATCAGCCTAGAGTTGCAACTAG
          3940      3950      3960      3970      3980      3990


        S   D   F   V   Q   I   E   G   C   D   V   L   F   V   N   A   T   V   S   D
       TTCTGACTTTGTTCAAATTGAAGGGTGCGATGTGCTGTTTGTTAATGCAACTGTAAGTGA
          4000      4010      4020      4030      4040      4050
```

DUPHAR INTERNATIONAL RESEARCH B.V.                    DIR 0389

```
   L  P  S  I  I  P  D  Y  I  D  I  N  Q  T  V  Q  D  I  L  E
TTTGCCTAGTATTATACCTGATTATATTGATATTAATCAGACTGTTCAAGACATATTAGA
     4060      4070      4080      4090      4100      4110


   N  F  R  P  N  W  T  V  P  E  L  T  F  D  I  F  N  A  T  Y
AAATTTTAGACCAAATTGGACTGTACCTGAGTTGACATTTGACATTTTTAACGCAACCTA
     4120      4130      4140      4150      4160      4170


   L  N  L  T  G  E  I  D  D  L  E  F  R  S  E  K  L  H  N  T
TTTAAACCTGACTGGTGAAATTGATGACTTAGAATTTAGGTCAGAAAAGCTACATAACAC
     4180      4190      4200      4210      4220      4230


   T  V  E  L  A  I  L  I  D  N  I  N  N  T  L  V  N  L  E  W
CACTGTAGAACTTGCCATTCTCATTGACAACATTAACAATACATTAGTCAATCTTGAATG
     4240      4250      4260      4270      4280      4290


   L  N  R  I  E  T  Y  V  K  W  P  W  Y  V  W  L  L  I  G  L
GCTCAATAGAATTGAAACCTATGTAAAATGGCCTTGGTATGTGTGGCTACTAATAGGCTT
     4300      4310      4320      4330      4340      4350


   V  V  I  F  C  I  P  L  L  L  F  C  C  C  S  T  G  C  C  G
AGTAGTAATATTTTGCATACCATTACTGCTATTTTGCTGTTGTAGTACAGGTTGCTGTGG
     4360      4370      4380      4390      4400      4410


   C  I  G  C  L  G  S  C  C  H  S  I  C  S  R  R  Q  F  E  N
ATGCATAGGTTGTTTAGGAAGTTGTTGTCACTCTATATGTAGTAGAAGACAATTTGAAAA
     4420      4430      4440      4450      4460      4470


   Y  E  P  I  E  K  V  H  V  H  *  I  *  N  V  N  S  I  I  C
TTACGAACCAATTGAAAAAGTGCACGTCCATTAAATTTAAAATGTTAATTCTATCATCTG
     4480      4490      4500      4510      4520      4530


   Y  N  S  S  C  F  C  *  R  I  L  L  R  M  M  N  K  V  F  K
CTATAATAGCAGTTGTTTCTGCTAGAGAATTTTGTTAAGGATGATGAATAAAGTCTTTAA
     4540      4550      4560      4570      4580      4590


   N  *  T
GAACTAAACT
     4600
```

DUPHAR INTERNATIONAL RESEARCH B.V.                    DIR 0389

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | ADVANCES IN EXPERIMENTAL MEDICINE AND BIOLOGY, vol. 185, no. 3, 1985, pages 63-82; S. HU et al.: "Studies of TGEV spike protein GP195 expressed in E. coli and by a TGE-vaccinia virus recombinant" * Whole article * | 1-9 | C 12 N 15/00 A 61 K 39/225 C 12 P 21/02 C 12 N 1/20 |
| X,D | JOURNAL OF VIROLOGY, vol. 57, no. 3, March 1986, pages 1010-1015, American Society for Microbiology; L. JACOBS et al.: "Characterization and translation of transmissible gastroenteritis virus mRNAs" * Page 1014 * | 2,3 | |
| A | IDEM | 1,5-9 | |
| A | US-A-4 335 105 (P.M. GOUGH) * Whole document * | 2-4 | |
| X,P | THE JOURNAL OF GENERAL VIROLOGY, vol. 68, no. 7, July 1987, pages 1883-1890, SGM; D. RASSCHAERT et al.: "The predicted primary structure of the peplomer protein E2 of the porcine coronavirus transmissible gastroenteritis virus" * Whole article, especially figure 2 * | 1-9 | TECHNICAL FIELDS SEARCHED (Int. Cl.4) C 12 N A 61 K C 12 P |

The present search report has been drawn up for all claims

| Place of search | Date of completion of the search | Examiner |
|---|---|---|
| THE HAGUE | 14-04-1988 | CUPIDO M. |